# FASCICULE DE BREVET EUROPEEN

(11) **EP 0 798 290 B1**
(45) Date de publication et mention de la délivrance du brevet: **16.02.2000**
(21) Numéro de dépôt: 97400568.8
(22) Date de dépôt: 14.03.1997
(51) Int. Cl.: C07C 249/06, C07C 251/44

(54) **Procédé de préparation de l'oxime de la cyclododécanone par photonitrosation de cyclododécane en présence de trichloronitrosométhane**
Verfahren zur Herstellung des Oxims von Cyclododecanon durch Photonitrosation von Cyclododecan in Gegenwart von Trichlornitrosomethan
Process for the preparation of the oxim of cyclododecanone by photonitrosation of cyclododecane in the presence of trichloronitrosomethane

(30) Priorité: 25.03.1996 FR 9603687
(43) Date de publication de la demande: 01.10.1997
(73) Titulaire: ELF ATOCHEM S.A., 92800 Puteaux, Hauts-de-Seine (FR)
(72) Inventeur: Ollivier, Jean, 64260 Arudy (FR)

(56) Documents cités:
- DE-A- 2 001 331
- FR-A- 2 094 612
- GB-A- 1 136 747
- CHEMICAL ABSTRACTS, vol. 76, no. 21, 22 Mai 1972 Columbus, Ohio, US; abstract no. 126097z, page 424; XP002019960 & TETRAHEDRON LETT., no. 7, 1972, pages 593-596, B.C. GOWENLOCK ET AL:

## Description

La présente invention a trait au domaine des polymères du type polyamide-12 obtenus par polymérisation du lauryllactame. Elle concerne plus particulièrement un procédé de préparation de l'oxime de cyclododécanone (intermédiaire réactionnel du lauryllactame) par photonitrosation de cyclododécane en présence de trichloronitrosométhane. Le lauryllactame est largement utilisé en tant que monomère pour la préparation de polyamide 12 par polymérisation. De nombreuses méthodes pour sa préparation sont décrites dans la littérature (voir par exemple "Procédés de Pétrochimie", tome 2, pp. 316-322, Edition Technip, 1986).

Les procédés industriels les plus connus sont ceux qui ont été développés par Hüls et Ato Chimie à partir de cyclododécane, Snia Viscosa à partir de mono-ozonide du cyclododécatriène, et UBE à partir de cyclododécanone.

Plus précisément, on obtient le lauryllactame selon le procédé proposé par Ato Chimie en deux étapes :
- dans la première étape, on forme l'oxime de la cyclododécanone par photonitrosation du cyclododécane selon la réaction suivante : 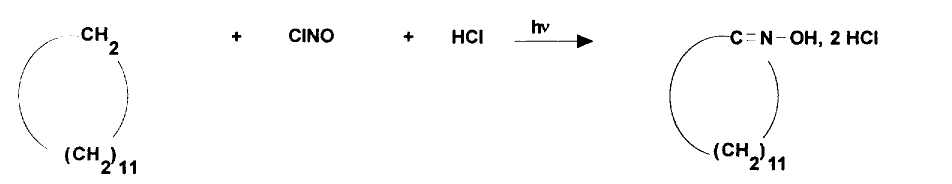
- et, dans la deuxième étape, l'oxime ainsi formée est soumise à une transposition de Beckmann en présence d'acide sulfurique

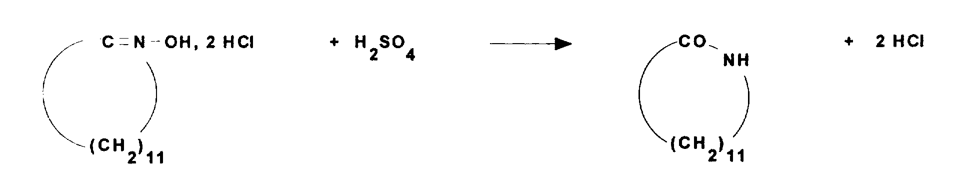

Dans la première étape, on réalise la photonitrosation en présence de chlorure de nitrosyle, lequel composé s'avère être également un agent chlorant du cyclododécane. On forme ainsi des sous-produits chlorés, tels que des chloroalcanes et des chloro-oximes. De tels composés, non valorisables, sont préjudiciables aussi bien au rendement et à la sélectivité de la réaction qu'à la qualité du produit final et doivent donc être éliminés par le biais de purifications longues et coûteuses.

Il a maintenant été trouvé que l'emploi de trichloronitrosométhane comme agent nitrosant permet, tout en gardant les avantages liés à la photonitrosation, d'améliorer la sélectivité en prévenant la formation de sous-produits chlorés précités.

L'invention a donc pour objet un nouveau procédé de préparation de l'oxime de la cyclododécanone, par photonitrosation de cyclododécane en présence d'acide chlorhydrique, caractérisé en ce que l'agent nitrosant est le trichloronitrosométhane.

Le trichloronitrosométhane du procédé selon l'invention est généralement formé à partir de chlorure de nitrosyle et de chloroforme par réaction photochimique conventionnelle, par exemple dans un réacteur comportant des lampes à vapeur de mercure ou de sodium. La réaction photochimique est généralement effectuée à une température comprise entre 10 et 20°C, à la pression atmosphérique. La concentration en poids de chlorure de nitrosyle dans le chloroforme est de préférence comprise entre 2 et 10 g/l.

On utilise le trichloronitrosométhane sous forme d'une solution dans les solvants chlorés, de préférence le chloroforme.

La concentration en poids de trichloronitrosométhane dans le solvant précité est généralement comprise entre 0,2 et 20 g/l et de préférence entre 8 et 12 g/l.

L'acide chlorhydrique est généralement mis en oeuvre sous forme d'acide chlorhydrique gazeux et anhydre.

Le cyclododécane peut être obtenu selon des méthodes connues de l'homme du métier, par exemple par cyclotrimérisation du butadiène et hydrogénation du cyclododécatriène formé.

Le procédé selon l'invention est mis en oeuvre par transformation directe du mélange constitué par le cyclododécane, l'acide chlorhydrique et le trichloronitrosométhane dans des conditions photochimiques conventionnelles.

Dans le but de faciliter l'extraction de l'oxime lors de la phase ultérieure de décantation, on ajoute avantageusement au mélange une faible quantité d'acide sulfurique dont le titre est compris entre 75 et 98 % en poids.

Le mélange est généralement irradié à une température comprise entre 0 et 30°C au moyen de lampe(s) à vapeur de mercure ou de sodium émettant entre 400 et 600 nm et de préférence 500 et 600 nm.

Le procédé selon l'invention est généralement mis en oeuvre à la pression atmosphérique.

A l'issue de la réaction, on soumet le mélange à une décantation et on récupère l'oxime de la cyclododécanone dans la phase aqueuse.

L'oxime ainsi formée peut être utilisée pour la préparation du lauryllactame, par exemple par transposition de Beckmann en présence d'acide sulfurique.

Les exemples suivants illustrent l'invention sans toutefois la limiter.

### EXEMPLE

On utilise un réacteur photochimique équipé d'un porte-lampe central à immersion et d'une lampe à vapeur de sodium à moyenne pression de 250 Watts émettant un maximum de radiations au voisinage de 595 nm. Le porte-lampe et la lampe sont thermostatés par une circulation d'eau à 15°C.

Ainsi équipé, le réacteur a un volume utile de 250 ml et la lampe présente un trajet optique égal à 6,5 mm.

L'introduction des réactifs est réalisée à la pression atmosphérique au moyen d'une tubulure équipée d'un fritté (porosité 4) située dans la partie inférieure du réacteur.

Les effluents gazeux issus du réacteur sont, après circulation dans un dispositif réfrigéré (température 0°C), dirigés vers un barboteur contenant une solution de soude relié à une torche.

### a) préparation du trichloronitrosométhane

Dans le réacteur, on introduit 200 ml de chloroforme dans lequel on dissout progressivement le chlorure de nitrosyle jusqu'à ce que la concentration soit égale à 2 g/l. On allume la lampe et on irradie pendant 3 heures tout en maintenant un débit constant de 2 l/h en chlorure de nitrosyle.

Après extinction de la lampe et arrêt de l'injection de chlorure de nitrosyle, on place le mélange obtenu sous un courant d'azote afin d'éliminer le chlorure de nitrosyle qui n'a pas réagi. On observe alors l'apparition d'une couleur bleue caractéristique du trichloronitrosométhane (présence d'une bande à 592 nm ; ε : 5,57 mole⁻¹. litre . cm⁻¹).

La concentration en trichloronitrosométhane dans le chloroforme est égale à 10 g/l.

### b) photonitrosation du cyclododécane

La solution chloroformique de trichloronitrosométhane précitée, à laquelle on ajoute 25 ml d'acide sulfurique à 85 %, 2 g d'acide chlorhydrique gazeux et 17 g de cyclododécane, est irradiée pendant 90 minutes sous agitation au moyen d'une pompe (renouvellement du volume du réacteur : 100 fois par heure).

A l'issue de la réaction, le mélange obtenu, qui a perdu sa couleur bleue, est décanté.

La phase aqueuse récupérée contient 2,59 g d'oxime de la cyclododécanone (soit un rendement de 97,7 % calculé sur la base du trichloronitrosométhane engagé) et aucune chloro-oxime.

La phase organique résiduelle ne contient pas de dérivé chloré du cyclododécane de départ.

### EXEMPLE COMPARATIF

On opère dans le réacteur décrit à l'exemple précédent, l'agent nitrosant utilisé étant le chlorure de nitrosyle.

Dans le réacteur contenant 200 ml d'une solution de cyclododécane dans le chloroforme (300 g/l) et 20 ml d'acide sulfurique à 85 % en poids, on injecte en continu du chlorure de nitrosyle gazeux de telle sorte que, après l'allumage de la lampe, la concentration dans le milieu réactionnel soit égale à 2 g/l.

Le milieu réactionnel, agité au moyen d'une pompe externe (renouvellement du volume du réacteur : 100 fois par heure) est irradié pendant 2 heures.

A l'issue de la réaction, le mélange réactionnel est décanté.

Dans la phase aqueuse, on récupère l'oxime de la cyclododécanone (sélectivité molaire : 88,35 %) ainsi que des chloro-oximes (5,4 % en poids du mélange).

La phase organique contient, outre le cyclododécane résiduel, des dérivés mono- et dichlorés du cyclododécane respectivement 4,95 et 1,65 % en poids du cyclododécane de départ.

## Revendications

1. Procédé de préparation d'oxime de la cyclododécanone par photonitrosation de cyclododécane en présence d'acide chlorhydrique et d'un agent nitrosant, caractérisé en ce que l'agent nitrosant est le trichloronitrosométhane.

2. Procédé selon la revendication 1 caractérisé en ce que le trichloronitrosométhane est en solution dans le chloroforme.

3. Procédé selon la revendication 2 caractérisé en ce que la concentration en poids de trichloronitrosométhane est comprise entre 0,2 et 20 g/l.

4. Procédé selon l'une des revendications 1 à 3 caractérisé en ce que la longueur d'onde des rayonnements lumineux est comprise entre 400 et 600 nm.

5. Procédé selon l'une des revendications 1 à 4 caractérisé en ce que la température de photonitrosation est comprise entre 0 et 30°C.

6. Procédé selon l'une des revendications 1 à 5 caractérisé en ce que l'on opère à pression atmosphérique.

7. Procédé selon l'une des revendications 1 à 6 caractérisé en ce que le trichloronitrosométhane est préparé par photonitrosation à partir de chlorure de nitrosyle et de chloroforme.

8. Procédé de préparation de lauryllactame dans lequel on prépare l'oxime de la cyclododécanone par photonitrosation de cyclododécane en présence d'acide chlorhydrique et de trichloronitrosométhane, et on soumet l'oxime formée à une transposition de Beckmann.

## Patentansprüche

1. Verfahren zur Herstellung von Cyclododecanonoxim durch photochemische Nitrosierung von Cyclododecan in Gegenwart von Salzsäure und einem Nitrosierungsmittel,
dadurch gekennzeichnet, daß
das Nitrosierungsmittel das Trichlornitrosomethan ist.

2. Verfahren nach Anspruch 1, dadurch gekennzeichnet, daß das Trichlornitrosomethan gelöst in Chloroform vorliegt.

3. Verfahren nach Anspruch 2, dadurch gekennzeichnet, daß die gewichtsbezogene Konzentration von Trichlornitrosomethan im Bereich von 0,2 bis 20 g/l liegt.

4. Verfahren nach einem der Ansprüche 1 bis 3, dadurch gekennzeichnet, daß die Wellenlänge der Strahlung im Bereich von 400 bis 600 nm liegt.

5. Verfahren nach einem der Ansprüche 1 bis 4, dadurch gekennzeichnet, daß die Temperatur bei der photochemischen Nitrosierung im Bereich von 0 bis 30 °C liegt.

6. Verfahren nach einem der Ansprüche 1 bis 5, dadurch gekennzeichnet, daß bei Atmosphärendruck gearbeitet wird.

7. Verfahren nach einem der Ansprüche 1 bis 6, dadurch gekennzeichnet, daß das Trichlornitrosomethan durch photochemische Nitrosierung aus Nitrosylchlorid und Chloroform hergestellt wird.

8. Verfahren zur Herstellung von Laurinlactam, wobei das Cyclododecanonoxim durch photochemische Nitrosierung von Cyclododecan in Gegenwart von Salzsäure und Trichlornitrosomethan hergestellt wird und das gebildete Oxim einer Beckmann-Umlagerung unterzogen wird.

## Claims

1. Process for preparing the oxime of cyclododecanone by photonitrozation of cyclododecane in the presence of hydrochloric acid and of a nitrozating agent, characterized in that the nitrozating agent is trichloronitrosomethane.

2. Process according to Claim 1, characterized in that the trichloronitrosomethane is in solution in chloroform.

3. Process according to Claim 2, characterized in that the concentration by weight of trichloronitrosomethane is between 0.2 and 20 g/l.

4. Process according to one of Claims 1 to 3, characterized in that the wavelength of the light radiation is between 400 and 600 nm.

5. Process according to one of Claims 1 to 4, characterized in that the photonitrozation temperature is between 0 and 30°C.

6. Process according to one of Claims 1 to 5, characterized in that it is carried out at atmospheric pressure.

7. Process according to one of Claims 1 to 6, characterized in that the trichloronitrosomethane is prepared by photonitrozation from nitrosyl chloride and chloroform.

8. Process for preparing lauryllactam in which the oxime of cyclododecanone is prepared by the photonitrozation of cyclododecane in the presence of hydrochloric acid and of trichloronitrosomethane, and the oxime formed is subjected to a Beckmann rearrangement.
